# EUROPEAN PATENT APPLICATION

(11) **EP 2 055 298 A1**
(43) Date of publication of application: **06.05.2009**
(21) Application number: 07119599.4
(22) Date of filing: 30.10.2007
(51) Int. Cl.: A61K 9/06, A61K 9/107, A61K 31/196

(54) **Topical composition**

(71) Applicant: NOVARTIS AG, 4056 Basel (CH)
(72) Inventor: The designation of the inventor has not yet been filed
(74) Representative: Liphardt, Bernd

(57) **Abstract**

The invention relates to beneficial topical pharmaceutical compositions comprising the diclofenac diethylammonium salt in unusually high amounts. Said compositions represent opaque emulsion-gels, in which diclofenac is kept fully dissolved.

## Description

The invention concerns topical formulations comprising the well-known and widely used non-steroidal anti-inflammatory drug (NSAID) diclofenac in emulsion-gel form. The currently commercially most successful product of this kind is Voltaren® Emulgel® comprising 1.16% diclofenac diethylamine salt (corresponding to 1% diclofenac sodium).

It was an object of the present invention to provide an emulsion-gel with similarly advantageous properties as Voltaren® Emulgel® but with even improved efficacy in conditions like e.g. back pain, osteoarthritis, or muscle or joint injuries, and allowing less frequent applications of the product per day.

It turned out quickly that said object could not be reached by simply increasing the concentration of diclofenac diethylammonium salt. When doing so, two main issues observed, among others, were an incomplete dissolution of the higher amounts of active substance used and an insufficient penetration through the skin of the higher amounts of active substance used. It was found that it is crucial for diclofenac to be fully dissolved (with no identifiable crystals of diclofenac being present in the formulation) rather than partly suspended. Only then a high, constant and reproducible permeation of diclofenac through the skin is guaranteed. A further issue, as it turned out, was that said improved topical formulation, as any commercial pharmaceutical product, had to be stable over time, that is to say have a long shelf life.

The inventors of the present invention ran a lot of experiments to test various diclofenac concentrations, various diclofenac salts and various additional excipients to resolve these and other issues. As an interim result, the following formulations were obtained (see Table 1):

**Table 1: Cumulative permeation in vitro of 20 mg/cm² of products on human skin**

| Time [h] | Voltaren® Emulgel® | Emulsion-gel 2.32% DEA¹⁾ 2% glycerin monolaurate | | Emulsion-gel 2.32% DEA¹⁾ 2% oleic acid | | Emulsion-gel 2.32% DEA¹⁾ 2% oleyl alcohol | |
|---|---|---|---|---|---|---|---|
| | mean | mean | n-fold increase | mean | n-fold increase | mean | n-fold increase |
| 0 | 0.00 | 0.00 | 0.00 | 0.00 | 0.00 | 0.00 | 0.00 |
| 2 | 0.30 | 0.53 | 1.77 | 0.35 | 1.16 | 0.36 | 1.22 |
| 4 | 0.83 | 1.26 | 1.51 | 0.75 | 0.90 | 1.18 | 1.42 |
| 8 | 1.73 | 3.80 | 2.19 | 3.49 | 2.02 | 3.82 | 2.21 |
| 24 | 6.65 | 13.51 | 2.03 | 20.47 | 3.08 | 18.33 | 2.76 |
| 32 | 8.81 | 18.40 | 2.09 | 28.19 | 3.20 | 24.11 | 2.74 |

| | | | | | | | |
|---|---|---|---|---|---|---|---|
| ¹⁾ DEA = diclofenac diethylammonium salt | | | | | | | |

Three emulsion-gels similar to Voltaren® Emulgel® but each including 2.32% of diclofenac diethylammonium salt and further 2% of either glycerin monolaurate, oleic acid or oleyl alcohol, turned out to include the active diclofenac in fully dissolved state and, moreover, provided a dramatically increased permeation of diclofenac through the skin, both as desired.

However, when examining said three formulations further, it turned out that the formulation containing 2 % of glycerin monolaurate was not suitable, as it did not show sufficient stability of the emulsion over time. What the formulation containing 2% of oleic acid was concerned, it did not have a sufficiently high viscosity, even when maximum possible amounts of gelling agents (carbomers) were applied. Moreover, when its microscopic aspect was evaluated, it turned out that the droplets forming the emulsion were too big, which bears the risk of an undesired separation of the two phases of the emulsion (unmixing). What is desired and needed is to have fine droplets upon microscopic inspection.

A further hurdle to overcome before completing the invention was local skin tolerance: The local skin tolerance of Voltaren® Emulgel® is known to be very good, i.e. the appearance of skin irritations after application is very rare, and also the systemic toxicity of said product is very low. A goal for the emulsion-gel of the present invention was to come as close as possible to the safety profile set by Voltaren® Emulgel®.

Therefore the invention relates to a topical pharmaceutical composition, which is in the form of an opaque emulsion-gel, and which comprises
(a) 1.2-4% (w/w) of diclofenac diethylammonium salt,
(b) 0.5-2% (w/w) oleyl alcohol,
(c) at least 40% (w/w) of water,
(d) 10-30% (w/w) of at least one C2-C4-alkanol,
(e) 3-15% (w/w) of at least one glycol solvent selected from the group consisting of 1,2-propanediol and polyethylene glycol (200-20000),
(f) 0.5-5% (w/w) of at least one gelling agent selected from the group consisting of carbomers,
(g) 2-8% (w/w) of at least one liquid lipid forming the oily phase of the emulsion-gel,
(h) 1-3% (w/w) of at least one non-ionic surfactant, and
(i) a basic agent to adjust the pH of the total composition to 6-9.

Preferably, diclofenac diethylammonium salt (a) is the only pharmaceutically active substance being present in the composition.

All percentages given are percentages by weight (w/w), if not indicated otherwise.

Preferred are those compositions that comprise
(a) 1.5-3.5% of diclofenac diethylammonium salt,
(b) 0.5-1.5% oleyl alcohol,
(c) 45-75% of water,
(d) 10-30% of ethanol, isopropanol, or mixtures thereof,
(e) 3-12% of 1,2-propanediol,
(f) 0.7-3% of at least one gelling agent selected from the group consisting of carbomers,
(g) 3-7% of at least one liquid lipid forming the oily phase of the emulsion-gel,
(h) 1-3% of at least one non-ionic surfactant, and
(i) 0.5-2% of diethylamine to adjust the pH of the total composition to 6.5-8.5.

Preferably, diclofenac diethylammonium salt (a) is present in amount of from 1.3 up to 3.5% - more preferably of from 1.5 up to 3.5%, especially of from 1.7 up to 3% and in particular of from 2 up to 2.5% - of the total composition.

Oleyl alcohol (b) is preferably present in an amount of from 0.5 up to 1.5% - more preferably of from 0.5 up to 1.3% - especially of from 0.6 up to 1.2% and in particular of from 0.7 up to 1% - of the total composition.

Water (c) is preferably present in an amount of from 45 up to 75%, preferably of from 50 up to 75%, especially of from 55 up to 75% and in particular of from 60 up to 70%, of the total composition.

Preferred as C2-C4-alkanols (d) are ethanol, isopropanol, or mixtures thereof; and in particular isopropanol. Typically, (d) is present in an amount of from 10 up to 30%, preferably of from 10 up to 25%, especially of from 12 up to 23% and in particular of from 15 up to 20%, of the total composition.

Preferably, the glycol solvent (e) is 1,2-propanediol. Alternatively, polyethylene glycol (200-20000), e.g. polyethylene glycol (200-1000), may also be used as (e). The glycol solvent (e) is preferably present in an amount of from 3 up to 12%, more preferably of from 3 up to 10%, especially of from 3 up to 8%, more especially of from 3 up to 7%, and in particular of from 3.5 up to 6%, of the total composition.

Carbomers (f), in the context of the present invention, are defined as homo- or copolymers of acrylic acid that are cross-linked, e.g. with an allyl ether of pentaerythritol (allyl pentaerythritol) or an allyl ether of sucrose (allyl sucrose). Copolymers are formed e.g. with minor levels of long chain alkyl acrylate co-monomers. Homopolymers are preferred. Especially preferred are carbomers 980, 940, 981, 941, 974, 934 and 910. In particular preferred are the following products provided by Noveon, Inc, Cleveland, Ohio, USA (formerly B F Goodrich): Carbopol® 980 and Carbopol® 974 - especially Carbopol® 980 -, and analogous carbomer products from other suppliers. Preferably, (f) is present in an amount of from 0.7 up to 3%, more preferably of from 0.8 up to 2%, especially of from 0.9 up to 1.8% and in particular of from 0.9 up to 1.6%, of the total composition.

The liquid lipid (g) forms the oily phase of the emulsion-gels of the invention. It can be of a vegetable or animal nature, or partly or completely synthetic. There come into consideration lipids without ester linkages, e.g. hydrocarbons, fatty alcohols or fatty acids, and lipids having ester linkages, e.g. glycerides - i.e. fatty acid esters of glycerol -, especially triglycerides, or esters of fatty acids, e.g. with C1-C36-alkanols, especially C8-C36-alkanols. Hydrocarbons are e.g. paraffin or petroleum jelly. Fatty alcohols are e.g. decanol, dodecanol, tetradecanol, hexadecanol or octadecanol. Fatty acids are e.g. C6-C24-, especially C10-C18-, alkanoic acids, e.g. hexanoic acid, octanoic acid, decanoic acid, dodecanoic acid, tetradecanoic acid, hexadecanoic acid or octadecanoic acid. Fatty acids further include unsaturated fatty acids, e.g. oleic acid or linoleic acid. Glycerides are e.g. olive oil, castor oil, sesame oil, it being possible for all said oils also to be hydrogenated; caprylic/capric acid triglyceride or glycerol mono-, di- and tri-esters with palmitic and/or stearic acid. Esters of fatty acids with C1-C36-alkanols are e.g. beeswax, carnauba wax, cetyl palmitate, lanolin, isopropyl myristate, isopropyl stearate, oleic acid decyl ester, ethyl oleate, or C6-C12-alkanoic acid esters - especially caprylic/capric acid esters - with saturated fatty alcohols, especially C12-C18 saturated fatty alcohols.

Preferably, the liquid lipid (g) comprises C6-C12-alkanoic acid C12-C18-alkyl esters. In particular preferred is a mixture of liquid paraffin and C6-C12-alkanoic acid C12-C18-alkyl esters - especially caprylic/capric acid esters with C12-C18 saturated fatty alcohols (coco-caprylate/caprate, e.g. Cetiol^{®}LC). The liquid lipid(s) (g) are preferably present in a total amount of from 3 up to 7%, especially of from 4 up to 6%, of the total composition. If a mixture of paraffin and coco-caprylate/caprate is used, the amount of paraffin is preferably 1.5-3%, especially 2-2.8%, and the amount of coco-caprylate/caprate is preferably 1.5-3%, especially 2-2.8%, of the total composition.

(h) A non-ionic surfactant is e.g. an ester of a fatty acid, especially a C8-C18 fatty acid, with monohydroxy or, preferably, polyhydroxy compounds, e.g. ethylene glycol, glycerol, anhydrosorbitol or pentaerythritol. Another important group of non-ionic surfactants is represented by the poly(oxyethylated) surfactants, which mean compounds that have at least one active hydrogen, e.g. fatty alcohols - especially a C8-C18 fatty alcohol -, fatty acids - especially a C8-C18 fatty acid -, sorbitan fatty acid esters, C1-C18-alkylphenols or C8-C18-alkylamines, and that all are poly(oxyethylated), preferably with from 2 up to 40 ethylene glycol or ethylene oxide units.

Examples for the above mentioned non-ionic surfactants are partial glycerin fatty acid esters, such as glycerin monostearate; partial fatty acid esters of sorbitan or polyoxyethylene sorbitan, such as sorbitan monolaurate or polyethylene glycol (5 to 20) sorbitan monostearate or monooleate; polyoxyethylene (3 to 40) fatty alcohol ethers, such as polyoxyethylene (3 to 12) lauryl ethers or polyoxyethylene (5 to 40) cetostearyl ethers; polyoxyethylene fatty acid esters, such as polyoxyethylene (8 to 100) stearate; polyoxyethylene C4-C12-alkylphenyl ethers, e.g. polyoxyethylene (nonyl or octyl)phenyl ethers; or polyoxyethylene C8-C18-alkylamines, e.g. polyoxyethylene oleylamine. Preferred are polyoxyethylene (10 to 30) fatty alcohol ethers, in particular polyoxyethylene (20) cetostearyl ether (e.g. Cetomacrogol 1000). Typically, the non-ionic surfactant (g) is present in an amount of from 1 up to 3%, preferably of from 1.5 up to 2.5%, of the total composition.

The basic agent (i) to adjust the pH of the total composition to 6-9 - especially 6.5-8 - is preferably diethylamine. In case that diethylamine is used, it is e.g. present in an amount of 0.5-2%, especially 0.7-1.6%, of the total composition. In general, (i) can be present e.g. in amount of from 0.1 up to 10% of the total composition.

The compositions of the inventions may optionally include further routine excipients known in the art, for example fragrances/perfumes, antioxidants, e.g. butylhydroxytoluene, antimicrobial preservatives, e.g. benzyl alcohol, benzalkonium chloride or parabens (= C1-C7-alkyl esters of 4-hydroxybenzoic acid, e.g. methyl 4-hydroxybenzoate), and coloring agents.

The topical pharmaceutical compositions according to the invention are administered in a manner known per se. For example, they are applied e.g. once, twice, or three or four times daily to the affected portions of the skin.

The invention further relates to a method of treating inflammatory diseases which comprises topically administering to a mammal in need of such treatment a therapeutically effective amount of one of the topical pharmaceutical compositions as specified herein above and below.

The manufacture of the topical pharmaceutical preparations is effected in a manner known per se, for example as described in the examples below.

The following examples are intended to illustrate the invention.

### Example 1: An emulsion gel comprising 2.32% of diclofenac diethylammonium salt

| Ingredients | | Amount (kg/100kg) |
|---|---|---|
| (a) | Diclofenac diethylammonium salt | 2.32 |
| (b) | Oleyl alcohol | 0.75 |
| (c) | Purified water | 64.26 |
| (d) | Isopropanol | 17.50 |
| (e) | 1,2-Propanediol (= Propylene glycol) | 5.00 |
| (f) | Carbomer 980 | 1.70 |
| (g') | Paraffin, liquid | 2.50 |
| (g") | Coco-caprylate/caprate | 2.50 |
| (h) | Polyoxyethylene-20-cetostearyl ether | 2.00 |
| (i) | Diethylamine | 1.35 |
| Butylhydroxytoluene (BHT) | | 0.02 |
| Perfumes | | 0.10 |
| | | 100.0 |

Manufacture: (a) is dissolved in (d), (e) and part of (c). Said solution is added to a mixture of the remainder of (c) and (f) which is neutralized by adding (i). All remaining components - (g'), (g") and (h), (b), BHT and perfumes - are heated and slowly added to the former mixture. Upon mixing a homogeneous emulsion-gel is obtained.

### Example 2: An emulsion gel comprising 3.48% of diclofenac diethylammonium salt

| Ingredients | | Amount (kg/100kg) |
|---|---|---|
| (a) | Diclofenac diethylammonium salt | 3.48 |
| (b) | Oleyl alcohol | 1.00 |
| (c) | Purified water | 49.35 |
| (d) | Isopropanol | 25.00 |
| (e) | 1,2-Propanediol | 10.00 |
| (f) | Carbomer 980 | 2.00 |
| (g') | Paraffin, liquid | 2.50 |
| (g") | Coco-caprylate/caprate | 2.50 |
| (h) | Polyoxyethylene-20-cetostearyl ether | 2.50 |
| (i) | Diethylamine | 1.55 |
| Butylhydroxytoluene | | 0.02 |
| Perfumes | | 0.10 |
| | | 100.0 |

### Manufacture: As described in Example 1.

Test example 1: The stability of the emulsion gel of Example 1 was tested via an assay of diclofenac diethylammonium salt. In doing so, the formulation was stored under various conditions (temperature/relative humidity) and for various storage times, and at the end the amount of diclofenac diethylammonium salt still being present was determined. The results were as follows:

| Storage time / Condition | 25°C/60% r.h. | 30°C/75% r.h. | 40°C/75% r.h. |
|---|---|---|---|
| 20g tubes: start | 100.1% | n.a. | n.a. |
| 20g tubes: 3 months | 100.2% | 100.4% | 100.6% |
| 20g tubes: 6 months | 99.7% | 99.4% | 100.6% |
| 100g tubes: start | 100.6% | n.a. | n.a. |
| 100g tubes: 3 months | 100.8% | 100.5% | 100.9% |
| 100g tubes: 6 months | 100.6% | 100.4% | 100.4% |

| | | | |
|---|---|---|---|
| (n.a. = not applicable) | | | |

It was demonstrated that the active substance is completely stable even under demanding storage conditions for long periods of time.

Test example 2 (microscopic examination): The emulsion gel of Example 1 is examined under 100x magnification and scrutinized for the presence of any crystals of diclofenac diethylammonium salt. Absolutely no, not even tiny, crystals of diclofenac diethylammonium salt are observed. One only sees the very fine droplets of the emulsion.

Test example 3 local skin tolerance test in vivo (in rabbits, n = 6)]: After daily application for 4 hours during 28 consecutive days, the cutaneous tolerance of the emulsion gel of Example 1 proved to be very good.

## Claims

1. A topical pharmaceutical composition, which is in the form of an opaque emulsion-gel, and which comprises
(a) 1.2-4% (w/w) of diclofenac diethylammonium salt,
(b) 0.5-2% (w/w) oleyl alcohol,
(c) at least 40% (w/w) of water,
(d) 10-30% (w/w) of at least one C2-C4-alkanol,
(e) 3-15% (w/w) of at least one glycol solvent selected from the group consisting of 1,2-propanediol and polyethylene glycol (200-20000),
(f) 0.5-5% (w/w) of at least one gelling agent selected from the group consisting of carbomers,
(g) 2-8% (w/w) of at least one liquid lipid forming the oily phase of the emulsion-gel,
(h) 1-3% (w/w) of at least one non-ionic surfactant, and
(i) a basic agent to adjust the pH of the total composition to 6-9.

2. A composition according to claim 1, which comprises
(a) 1.5-3.5% of diclofenac diethylammonium salt,
(b) 0.5-1.5% oleyl alcohol,
(c) 45-75% of water,
(d) 10-30% of ethanol, isopropanol, or mixtures thereof,
(e) 3-12% of 1,2-propanediol, ,
(f) 0.7-3% of at least one gelling agent selected from the group consisting of carbomers,
(g) 3-7% of at least one liquid lipid forming the oily phase of the emulsion-gel,
(h) 1-3% of at least one non-ionic surfactant, and
(i) 0.5-2% of diethylamine to adjust the pH of the total composition to 6.5-8.5.

3. A composition according to claim 1 or claim 2, wherein the oleyl alcohol (b) is present in an amount of from 0.6 up to 1.2% of the total composition.

4. A composition according to any one of claims 1-3, wherein the component (d) is isopropanol.

5. A composition according to any one of claims 1-4, wherein the liquid lipid (g) is selected from the group consisting of hydrocarbons, fatty alcohols, fatty acids, glycerides, esters of fatty acids and any mixtures thereof.

6. A composition according to any one of claims 1-5, wherein the non-ionic surfactant (h) is selected from the group consisting of esters of fatty acids with monohydroxy or polyhydroxy compounds and poly(oxyethylated) surfactants and any mixtures thereof.
